# FASCICULE DE BREVET EUROPEEN

(11) **EP 1 140 986 B1**
(45) Date de publication et mention de la délivrance du brevet: **11.12.2002**
(21) Numéro de dépôt: 00900564.6
(22) Date de dépôt: 12.01.2000
(51) Int. Cl.: C07K 7/02

(54) **PSEUDOPEPTIDE, PROCEDE DE SYNTHESE, REACTIF ET APPLICATIONS**
PSEUDOPEPTID, VERFAHREN ZUR HERSTELLUNG, REAGENZ UND VERWENDUNGEN
PSEUDOPEPTIDE, SYNTHESIS METHOD, REAGENT AND APPLICATIONS

(30) Priorité: 15.01.1999 FR 9900597
(43) Date de publication de la demande: 10.10.2001
(73) Titulaire: BIO MERIEUX, 69280 Marcy l'Etoile (FR)
(72) Inventeur: BRIAND, Jean-Paul, F-67000 Strasbourg (FR); SEMETEY, Vincent, F-67000 Strasbourg (FR); LIMAL, David, F-67300 Schiltigheim (FR)
(74) Mandataire: Guerre, Dominique
(86) Numéro de dépôt international: FR0000053
(87) Numéro de publication internationale: WO00042065

(56) Documents cités:
- EP-A- 0 126 974
- WO-A-92/13883
- US-A- 5 475 013
- LIMAL D ET AL: "Solid-phase Synthesis of N,N'-Unsymmetrically Substituted Ureas: Application to the Synthesis of Carbaza Peptides" TETRAHEDRON LETTERS, vol. 40, no. 14, 2 avril 1999 (1999-04-02), page 2749-2752 XP004159428 ISSN: 0040-4039
- C.G. BRADSHAW ET AL.: "Synthesis and characterisation of selective flourescent ligands for neurokinin NK2 receptor" J. MED. CHEM., vol. 37, 1994, pages 1991-1995, XP002117380
- DALLAIRE C ET AL: "Synthesis of new building blocks: towards the analogs of peptide nucleic acids (PNAs)<1a" TETRAHEDRON LETTERS, vol. 39, no. 29, 16 juillet 1998 (1998-07-16), page 5129-5132 XP004162398 ISSN: 0040-4039
- NOUVET A ET AL: "Synthesis of New Perhydro-(1,4)-diazepin-2-ones as Constrained Peptidomimetics" TETRAHEDRON LETTERS, vol. 39, no. 15, 9 avril 1998 (1998-04-09), page 2099-2102 XP004110641 ISSN: 0040-4039

## Description

Depuis des années de nombreuses équipes se sont attachées à synthétiser des analogues de peptides ou de protéines qui miment les activités biologiques des peptides ou protéines naturels. On peut citer à titre d'exemple les analogues peptidiques obtenus par remplacement d'un ou plusieurs acides aminés de la série L par un ou des acides aminés correspondants de la série D, les peptides présentant une modification au niveau d'au moins une des liaisons peptidiques, telles que les liaisons rétro, inverso, rétro-inverso, carba et aza.

La liaison carba (CH₂-CH₂) a été décrite comme un mime potentiel de la liaison peptidique (Mendre C. et al., European J. Pharmacol., 186, p213-222, 1990; Attwood et al., Bioorg. Med. Chem. Lett., 7, p429-432, 1997). Par ailleurs, le remplacement du carbone α par un atome d'azote partiel ou complet sur un peptide a permis d'obtenir des pseudopeptides intéressants dénommés azapeptides et azatides respectivement (Gante, J., Synthesis, p405-413, 1989 ; Han H. et Janda K.D., J. Amer. Chem. Soc, 118, p2539-2544, 1996).

D'une manière générale ces analogues peptidiques, dénommés pseudopeptides, présentent comme premier avantage une stabilité métabolique supérieure à celle des peptides ou protéines naturels en raison du fait qu'ils ne sont pas dégradés par les protéases naturelles ou le sont moins vite. Par ailleurs, les changements de conformation induits par ces modifications chimiques peuvent améliorer les propriétés biologiques de ces pseudopeptides, voir par exemple les analogues décapeptidiques antagonistes des hormones hypothalamiques décrits dans WO-A-92/13883.

Si les techniques de synthèse des peptides dits naturels, notamment sur support solide, sont bien rodées et permettent de préparer facilement des peptides comprenant plusieurs dizaines d'acides aminés, l'introduction de ces modifications pour préparer des pseudopeptides rend la synthèse plus complexe, en particulier pour des pseudopeptides longs.

Par ailleurs, dans le domaine de l'immunologie et aussi bien dans le diagnostic des maladies virales ou auto-immunes que dans l'immunothérapie ou la vaccination, les peptides synthétiques mimant les épitopes des protéines représentent une alternative de choix. La taille des peptides analogues de ces déterminants antigéniques ou épitopes est un facteur important dans le choix de ces peptides et a fait l'objet de nombreuses publications (M.H.V Regenmortel, Immunology Today, 10(8), p266-271, 1989 ou M.H.V Regenmortel, Biomedical Peptides, Proteins & Nucleic Acids, 1, p109-116, 1995). Si à l'origine, il était admis qu'un épitope comporte entre 15 et 22 acides aminés, les études récentes montrent que cette taille peut être réduite à quelques acides aminés. Dans le domaine de l'immunité, les études cristallographiques sur l'interaction des peptides et du complexe majeur d'histocompatibilité (CMH) indiquent une taille de 9 à 13 acides aminés pour une bonne interaction avec les molécules du CMH de classe I et de 9 à 25 pour le CMH de classe II (H.G. Rammensee, Current Opinion in Biotechnology, 7, p85-96, 1995). De même dans le diagnostic, la taille est un facteur critique pour l'utilisation des peptides. Dans le cas du VIH (virus d'immunodéficience humaine), les épitopes les plus petits comportent de 4 à 6 acides aminés mais les peptides utilisés possèdent toujours une taille supérieure d'au moins 12 acides aminés (D. Osmanov, AIDS, 5(1), WHO1-WHO9, 1991). Dans un autre exemple comme le diagnostic de la maladie de Chagas, les peptides utilisés comportent au minimum 12 acides aminés (WO-A-97/18475). Dans (Bradshaw C.G. et col., J. Med. Chem.,37,1991-1995,1994) des sondes fluorescentes analogues de l'antagoniste heptapeptidique de NK₂, ont été obtenues par substitution d'un acide aminé et couplage avec un fluorophore.

C'est l'objet de la présente invention que de décrire une nouvelle famille de pseudopeptides comportant un nouveau motif carbaza modifiant de manière significative le squelette peptidique et dont la mise en oeuvre dans le cadre de la synthèse de peptides soit aisée aussi bien en phase solide qu'en phase liquide et ce, même pour des peptides de taille importante et notamment supérieure à 6 acides aminés. Cette nouvelle famille de pseudopeptides est utilisable dans le domaine diagnostique pour fournir des méthodes de diagnostic in vitro de pathologies associées à la présence de protéines endogènes ou exogènes chez un individu, ou dans le domaine thérapeutique et notamment l'immunothérapie ou la vaccination. Ces pseudopeptides ont une taille d'au moins 6 acides aminés comprenant au moins un motif choisi parmi les motifs B de formule générale I et/ou II définies ci-dessous : 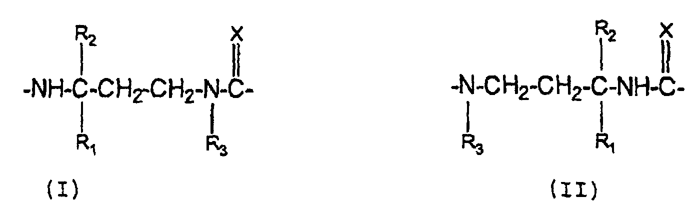 dans lesquelles :
R₁, R₂ et R ₃ représentent chacun indépendamment l'un de l'autre une chaîne latérale d'acides aminés et peuvent être identiques ou différents, et
X représente un atome d'oxygène ou de soufre, préférentiellement un atome d'oxygène.

Avantageusement R₂ représente un atome d'hydrogène.

Par acides aminés, on entend les acides aminés primaires qui codent pour les protéines, les acides aminés dérivés après action enzymatique comme la trans-4-hydroxyproline et les acides aminés naturels mais non présents dans les protéines comme la norvaline, la N-méthyl-L leucine, la staline (Hunt S. dans Chemistry and Biochemistry of the amino acids, Barett G.C., ed., Chapman and Hall, London, 1985), les acides aminés protégés par des fonctions chimiques utilisables en synthèse sur support solide ou en phase liquide et les acides aminés non naturels. Des exemples de ces acides aminés non naturels sont donnés dans le catalogue Novabiochem (Catalog & Peptide synthesis Handbook ; 1999 ; CH-4448, Lâufelfinfgen, Suisse) ou le catalogue Néosystem (Catalogue 1997/1998 ; 67100 Strasbourg, France).

Par chaîne latérale d'acides aminés, on entend l'ensemble des chaînes latérales des acides aminés tels que définis précédemment. Dans le cas de la proline, il est entendu que la chaîne latérale R₁ ou R₂ dans la formule du motif B se cyclise pour se lier à l'azote en alpha. De même, R₁ et R₂ peuvent se lier de manière covalente.

De préférence le pseudopeptide comporte au moins 9 acides aminés. Avantageusement dans le cas du diagnostic, le pseudopeptide comporte au moins 12 acides aminés. Le motif B tel que défini, représente 2 acides aminés puisque le squelette linéaire dudit motif B possède une structure à 6 atomes.

De préférence, la fonction NH de la formule I et la fonction NR₃ de la formule II sont liées à un groupe CX, et/ou la fonction CX des formules I et II sont liées à un groupe NH ou NR₃, lesdits groupes CX, NH et NR₃ appartenant à un motif peptidique ou pseudopeptidique.

L'invention concerne également un procédé de synthèse du pseudopeptide contenant au moins un motif B. Pour cela, la ou les molécules nécessaires sont des diamines monoprotégées de structure IIIa ou IIIb suivante : 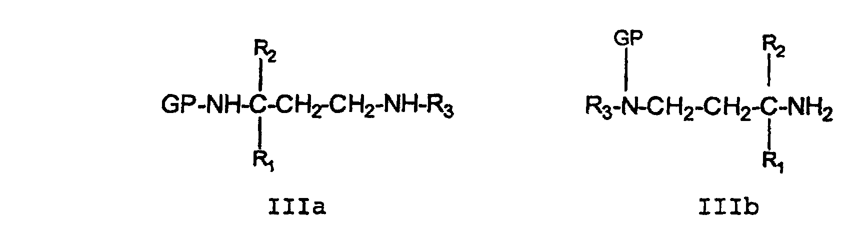 où
GP représente un groupe protecteur quelconque de fonction amine comme par exemple ceux décrits dans T.W. Greene and P.G.M. Wuts, Protective Groups in Organic Synthesis, 2^{ème} édition, John Wiley and Sons, New York, 1991 ; préférentiellement ceux couramment utilisés en synthèse peptidique à savoir :
Boc (tertiobutyloxycarbonyle),
Fmoc (9-fluorénylméthylèneoxycarbonyle),
Cbz (carboxybenzyle), ou
Alloc (allyloxycarbonyle), et
R₁, R₂ et R₃ représentent chacun indépendamment l'un de l'autre une chaîne latérale d'acides aminés et peuvent être identiques ou différents.

Cette molécule est ensuite couplée à une amine par l'intermédiaire d'un agent de carbonylation. A titre d'exemple, on peut citer le N,N'-carbonyldiimidazole (CDI) (Zhang, X.; Rodrigues, J.; Evans, L.; Hinckle, B.; Ballantyne, L.; Pena, M. J. Org. Chem. 62, 6420-6423, 1997), le carbamate de p-nitrophényle (Hutchins, S.M. & Chapman K.T. Tet Lett. 36, 2583-2586, 1995), le carbonate de 2,4-dinitrophényle (Quibell, M.; Turnell, W.G.; Johnson, T. J. Chem. Soc. Perkin Trans. I 2843-2849), carbonate de N,N'-disuccunimidyle (DSC) (Takeda, K.; Akagi, Y.; Saiki, A.; Tsukahara, T.; Ogura, H. Tet. Lett. 1983, 24, 4569-4572) et plus particulièrement le triphosgène (Majer, P. & Randad, R.S. J. Org. Chem. 59, 1937-1938, 1994). Cette réaction peut être effectuée sur un support solide ou en phase homogène.

Grâce à cette technique de couplage, le motif B peut être introduit à n'importe quelle position du pseudopeptide et il est aisé de préparer un pseudopeptide comportant plusieurs motifs B correspondant aux formules I et/ou II. Le pseudopeptide peut comporter exclusivement un enchaînement de motifs B correspondant aux formules I et/ou II.

Le pseudopeptide selon l'invention peut être modifié après ou pendant la synthèse comme par exemple par couplage avec des traceurs, des ligands ou anti-ligands, des protéines, des vitamines, par phosphorylation, sulfatation, glycosylation, hydroxylation. Les couples biotine / streptavidine, lectine / sucre, haptène / anticorps, chélatant / molécules chélatées, hormone / récepteur, polynucléotide / polynucléotide complémentaire sont des exemples des couples ligand / anti-ligand.

Un exemple de stratégie de modification d'un peptide par la biotine est donné dans Limal, D.; Briand, J.P.; Dalbon, P.; Jolivet, M., 1998, J. Peptide Res. 52, 121-129.

La structure du pseudopeptide peut subir des modifications comme des liaisons intrapeptidiques ou interpeptidiques. A titre d'exemples pour la formation de liaison intrapeptidique, la création de ponts disulfure entre différentes chaînes latérales de cystéines ou bien la formation de lactames entre deux chaînes latérales ou entre les deux extrémités C-terminale et N-terminale sont envisageables. Les liaisons interpeptidiques peuvent conduire à la formation de multimères peptidiques réticulés ou non par l'utilisation de réactifs de couplage bifonctionnels.

Des exemples de stratégie de couplage pour la modification du pseudopeptide sont donnés dans Chemistry of protein conjugation and cross-linking, Wong S. S., CRC press, Boca Raton, 1991 ou dans Bioconjugate techniques, Hermanson G.T., Academic Press, San Diego, 1996. Les pseudopeptides selon l'invention peuvent être linéaires cycliques ou branchés.

La synthèse du pseudopeptide peut être réalisée sur support solide par les techniques récurrentes classiques, par des techniques de ligation chimique (W. Lu et al, FEBS Letters, 429, p31-35, 1998 ou J.A Camarero et al, J. Peptide Res., 51, p303-316, 1998) ou par des techniques de condensation de fragment (Chemical approaches to the synthesis of peptides and proteins, Lloyd-Williams P., Albericio F., Giralt E., CRC press, Boca Raton, 1997) ou par combinaison de ces différentes techniques.

Un autre objet de l'invention est un réactif de détection d'une pathologie associée à la présence de protéines endogènes ou exogènes ledit réactif comprenant en outre un pseudopeptide de l'invention comme substance réactive. Le pseudopeptide est avantageusement marqué par un traceur ou la biotine. De préférence, la taille du pseudopeptide est d'au moins 12 acides aminés.

Les pathologies peuvent concerner toutes les pathologies animales ou humaines et notamment les pathologies humaines et en particulier les pathologies d'origine virale, parasitaire, le domaine du cancer, des maladies auto-immunes, des maladies neurodégénératives.

La détection de pathologies peut se faire de manière directe ou indirecte. Par direct, on entend la détection de cette pathologie dans un échantillon biologique provenant de l'organisme humain ou animal comme par exemple le sang, l'urine, le crachat, un frottis. Par indirect, on entend la détection de protéines dans des échantillons comme par exemple l'eau, l'air, les aliments, les produits pharmaceutiques, les cosmétiques qui peuvent entrer en contact avec ledit organisme humain ou animal pour provoquer une pathologie.

Un objet de l'invention est un kit de détection de pathologies associées à la présence de protéines endogènes ou exogènes comprenant le réactif décrit ci-dessus, fixé sur un support solide, immunologiquement compatible avec ledit réactif.

Le terme "support solide" tel qu'utilisé ici inclut tous les matériaux sur lesquels peut être immobilisée une molécule biologique pour une utilisation dans des tests diagnostiques et dans des processus de séparation. Des matériaux naturels ou de synthèse, modifiés ou non chimiquement, peuvent être utilisés comme support solide, notamment les polysaccharides tels que les matériaux à base de cellulose, par exemple du papier, des dérivés de cellulose tels que l'acétate de cellulose et la nitrocellulose, du dextran ; des polymères tels que polychlorures de vinyle, polyéthylènes, polystyrènes, polyacrylates, polyamides, ou des copolymères à base de monomères du type styrène, esters d'acides carboxyliques insaturés, chlorure de vinylidène, diènes ou composés présentant des fonctions nitrile (comme l'acrylonitrile) ; des copolymères chlorure de vinyle / propylène, chlorure de vinyle / acétate de vinyle ; des fibres naturelles telles que le coton et des fibres synthétiques telles que le nylon ; des matériaux inorganiques tels que la silice, le quartz des verres, des céramiques ; des latex, c'est-à-dire des dispersions aqueuses colloïdales d'un polymère quelconque insoluble dans l'eau; des particules magnétiques ; des dérivés métalliques, etc.

Le support solide peut être notamment sous la forme d'une plaque de microtitration, d'une feuille, d'un cône, d'un tube, de billes, de particules ou analogues.

La fixation du réactif peut être réalisée de manière directe ou indirecte.

De manière directe, deux approches sont possibles : soit par adsorption du réactif sur le support solide, soit par liaison covalente. Dans une variante, le pseudopeptide du réactif peut être couplé à un polypeptide, une protéine, un fragment d'acide nucléique pour améliorer la fixation sur la phase solide.

De manière indirecte, on peut fixer préalablement (par covalence ou adsorption) un anti-réactif capable d'interagir avec le réactif de façon à immobiliser l'ensemble sur le support solide. A titre d'exemple, la streptavidine adsorbée sur le support solide peut permettre la fixation d'un pseudopeptide portant une biotine ou bien un anticorps (monoclonal, polyclonal ou un fragment d'anticorps) dirigé contre le motif B de l'invention peut permettre cette même fixation du pseudopeptide.

L'invention concerne en outre un procédé de détection et/ou de dosage de molécules biologiques, et notamment d'anticorps, présentes dans un échantillon dans lequel on utilise le réactif selon l'invention pour former un complexe immun avec lesdites molécules biologiques si elles sont présentes dans l'échantillon.

L'invention concerne notamment un procédé pour la détection et/ou le dosage d'anticorps dans un échantillon comprenant les étapes consistant à mettre en contact ledit échantillon avec un réactif de l'invention dans des conditions permettant une réaction immunologique, puis à détecter et/ou doser le complexe immun éventuellement formé.

Dans un mode particulier, le réactif de l'invention est fixé sur la phase solide et le complexe immun est détecté à l'aide d'un deuxième anticorps marqué par un traceur.

Dans un autre mode particulier, le complexe immun entre le réactif marqué et la molécule biologique est formé en phase homogène et sa présence est détectée par une modification physico-chimique du traceur lié à la formation du complexe immun.

A titre d'exemple, ce deuxième anticorps est un anticorps monoclonal, polyclonal ou un fragment de type Fab, dirigé par exemple contre des anticorps humains dans le cas d'un échantillon biologique humain.

Par traceur, on entend une entité capable de générer un signal détectable.

Le traceur peut notamment être choisi parmi :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase.
- les chromophores comme les composés fluorescents, luminescents, colorants.
- les groupements à densité électronique détectable par microscopie électronique ou par leurs propriétés électriques comme la conductivité, l'ampérométrie, la voltamétrie, les mesures d'impédance.
- les groupements détectables par des méthodes optiques comme la diffraction, la résonance plasmon de surface, la variation d'angle de contact ou des méthodes physiques comme la spectroscopie de force atomique, l'effet tunnel.

Le marquage par un traceur peut être réalisé indifféremment de manière directe ou indirecte.

Par marquage direct, on entend la fixation covalente du traceur. Par marquage indirect, on entend la fixation non covalente du traceur notamment par des interactions ligand / anti-ligand.

L'invention concerne également un procédé pour la détection et/ou le dosage d'un antigène présent dans un échantillon par une technique de compétition dans lequel on met en contact, simultanément ou en deux étapes, ledit échantillon avec une quantité prédéterminée d'anticorps dirigé contre un partie de l'antigène et une quantité prédéterminée d'un réactif de l'invention, et on détermine la présence et/ou la quantité d'antigène présent dans ledit échantillon.

Dans un mode particulier, c'est l'anticorps qui est fixé sur la phase solide et le réactif de l'invention est marqué par un traceur.

L'invention concerne également un procédé pour la détection et/ou le dosage d'un anticorps présent dans un échantillon par une technique de compétition dans lequel on met en contact simultanément ledit échantillon avec une quantité prédéterminée d'antigène dont au moins une partie est reconnue par ledit anticorps et une quantité prédéterminée d'un réactif de l'invention, et on détermine la présence et/ou la quantité d'anticorps présent dans ledit échantillon.

Dans un mode de réalisation particulier, l'antigène est fixé sur la phase solide et le réactif de l'invention est marqué par un traceur. Dans une autre variante, le réactif est fixé sur la phase solide et l'antigène est marqué par un traceur.

Les pseudopeptides de l'invention présentent en outre un intérêt dans l'obtention de vaccins. Il est aujourd'hui acquis que les analogues peptidiques ont la capacité de stimuler les lymphocytes-T (P. Aichele et al. 1995. T cell priming versus T cell tolerance induced by synthetic peptide. J. Exp. Med. 182 :261 ; S. Tourdot et al. 1997. Chimeric peptides : a new approach to enhancing the immunogenicity of peptides with low MHC class I affinity : application in antiviral vaccination. J. Immunol. 159 :2391).

Ainsi, l'invention a également pour objet les anticorps dirigés contre les pseudopeptides selon l'invention qui peuvent être monoclonaux ou polyclonaux. Lesdits anticorps sont susceptible d'être obtenus par immunisation d'un animal avec au moins un pseudopeptide selon l'invention. Les anticorps selon l'invention sont plus particulièrement caractérisés en ce qu'ils sont susceptibles de former un complexe avec des pseudopeptides et/ou les protéines ou peptides parents correspondants à ces derniers.

Par protéine parent, on entend une protéine naturelle et par peptide parent, on entend
soit un peptide existant tel quel à l'état naturel, notamment dans un microorganisme supérieur et notamment l'organisme humain,
soit un peptide issu d'une protéine telle qu'elle existe à l'état naturel dans les organismes susmentionnés, notamment par fragmentation de ladite protéine ou par synthèse peptidique,
soit un peptide d'intérêt immunologique obtenu par synthèse peptidique,
soit un peptide issu d'une protéine telle qu'elle existe à l'état naturel mais dont l'activité immunologique a été modifiée, conservée ou optimisée par remplacement de certains aminoacides, comme par exemple à la suite d'un criblage d'une librairie de peptides analogues obtenue par synthèse peptidique.

Les anticorps anti-pseudopeptides de l'invention reconnaissent le peptide parent ou la protéine parente avec une affinité au moins égale à celle présentée par les anticorps anti-peptide parent ou anti-protéine parente vis à vis du peptide parent ou de la protéine parente. La constante d'affinité à l'équilibre Ka des complexes est un moyen de mesurer l'affinité.

L'invention concerne également les anti-idiotypes susceptibles d'être obtenus par immunisation d'un animal avec lesdits anticorps tels que définis ci-dessus.

Au cours d'études récentes, certains auteurs, dont ceux de la présente invention (J.P. Briand et al. 1997. A retro-inverso peptide corresponding to the GH loop of foot-and-mouth disease virus elicits high levels of long-lasting protective neutralizing antibodies. Proc. Natl. Sci. USA 94 :12545 ; C. Stemmer et al. 1999. Protection against lymphocytic choriomeningitis virus infection induced by a reduced peptide bond analogue of the H-2D^{b}-restricted CD8(+) T cel epitope GP33. J. Biol. Chem. 274 :5550), ont montré que des analogues peptidiques pouvaient avantageusement remplacer des peptides naturels, en thérapie. A titre d'exemple, il a été observé que les modifications du squelette peptidique peuvent considérablement influencer les interactions du complexe CMH / peptide avec le récepteur des lymphocytes-T (C. Stemmer et al. 1999. Protection against lymphocytic choriomeningitis virus infection induced by a reduced peptide bond analogue of the H-2D^{b}-restricted CD8(+) T cel epitope GP33. J. Biol. Chem. 274 :5550 ; M. Ostankovitch et al. 1998. A partially modified retro-inverso pseudopeptide modulates the cytokine profile of CTL specific for an influenza virus epitope. J. Immunol. 161 :200 ; S. Calbo et al. 1999. Role of peptide backbone in T cel recognition. J. Immunol. 162 :4657).

Une autre application des pseudopeptides selon l'invention est une composition thérapeutique active et notamment une composition immunothérapeutique active, préférentiellement une composition vaccinale comprenant à titre de principe actif un pseudopeptide présentant une demi-vie supérieure à celle des protéines naturelles ou à celle des peptides de synthèse issus ou non de ces protéines naturelles (ces protéines naturelles, ou ces peptides issus ou non de ces dernières étant désignés par l'expression protéines ou peptides parents) dont ils sont les analogues, ledit principe actif étant éventuellement sous la forme d'un conjugué ou d'un excipient pharmaceutiquement acceptable.

Les pathologies susmentionnées susceptibles d'être traitées dans le cadre de la présente invention sont principalement soit des maladies d'origine virale, bactérienne ou parasitaire, lorsqu'elles sont associées à la présence du microorganisme lui-même, soit des maladies auto-immunes lorsqu'elles sont associées à la présence de protéines ou peptides endogènes perturbant le fonctionnement physiologique normal d'un organisme lorsque ces derniers jouent directement un rôle d'anticorps ou induisent la formation d'anticorps reconnaissant et altérant des sites particuliers de l'organisme comme par exemple en formant des dépôts de complexes anticorps / antigènes ou en provoquant des états inflammatoires. Les pathologies susmentionnées peuvent être également être des maladies neurodégénératives lorsqu'elles sont associées à la présence dans l'organisme de protéines exogènes ayant pour effet de provoquer des lésions neurologiques. Les pseudopeptides utilisés pour la préparation des compositions pharmaceutiques ou des vaccins sont avantageusement des pseudopeptides dont le squelette est formé entièrement par un enchaînement de motifs B de formule générale I et/ou II.

L'invention vise plus particulièrement l'utilisation d'un pseudopeptide tel que défini ci-dessus, pour la préparation d'un vaccin dans le cadre de la prévention de pathologies associées à la présence dans l'organisme d'un individu d'une ou plusieurs protéine(s), exogène (s) ou endogène(s) susceptible(s) d'être reconnue(s) par des anticorps dirigés contre les pseudopeptides ou dirigés contre les anti-idiotypes selon l'invention.

L'invention vise également toute composition pharmaceutique comprenant au moins un pseudopeptide tel que défini ci-dessus ou au moins un anti-idiotype susmentionné, associé à une molécule porteuse protéique ou non, pouvant induire *in vivo* la production d'anticorps neutralisant lesdites protéines exogènes ou endogènes responsables de la pathologie, ou induire *in vivo* une réponse immune cellulaire cytotoxique. L'invention concerne en outre toute composition pharmaceutique comprenant au moins un anticorps défini ci-dessus.

S'agissant de l'utilisation des pseudopeptides dans le cadre de médicaments destinés au traitement des maladies auto-immunes, il convient de rappeler que la pathogénèse de nombreuses maladies auto-immunes implique la présentation d'autoantigènes (liés au molécules du CMH) au récepteur de cellules T autoréactives qui ont d'une façon ou d'une autre échappé au processus de tolérance du soi. Aussi le développement de nouvelles stratégies pour moduler la réponse des cellules T autoréactives pourrait conduire à des approches thérapeutiques capables de traiter certaines maladies auto-immunes.

Certaines maladies auto-immunes sont associées à des allèles CMH I ou II spécifiques. Ainsi l'utilisation de peptides bloquants capables d'interagir avec une molécule CMH donnée (par exemple une molécule CMH de classe II associée à une maladie auto-immune particulière) mais ne pouvant pas activer la réponse cellulaire T pathogénique est prometteuse. Cependant la dégradation des peptides dans les milieux biologiques rend leur utilisation difficile. Dans ce cas les pseudopeptides de par leur stabilité seraient très avantageux.

Les exemples qui suivent permettent d'illustrer quelques avantages de l'invention sans toutefois en limiter la portée. Ils font référence au dessin annexé dans lequel :
- la figure 1 illustre la synthèse d'une diamine chargée de mimer la séquence dipeptidique (Ala-Val) une fois introduite dans un peptide ; cette synthèse a été réalisée selon les deux stratégies utilisées couramment en synthèse peptidique (Boc et Fmoc) pour démontrer la généralité de la voie proposée ;
- la figure 2 illustre l'introduction sur support solide de l'amine monoprotégée via une carbonylation conduisant à l'isocyanate.

### Exemple 1 : Synthèse d'une diamine protégée pour l'introduction du motif B carbaza dans un pseudopeptide.

La voie de synthèse utilisée pour parvenir à ce dérivé d'acide aminé est la suivante.

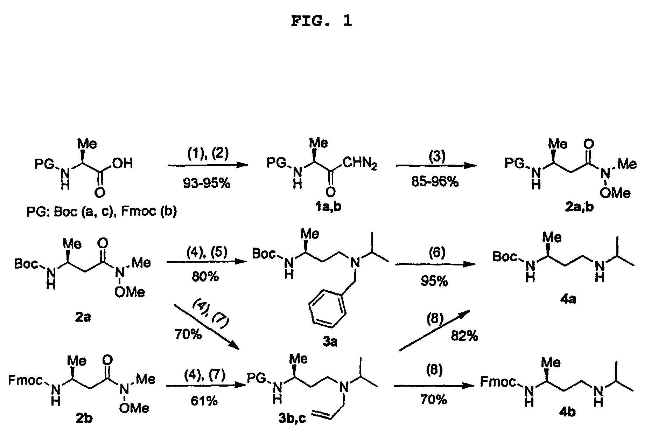

Un acide aminé N-protégé naturel ou non est tout d'abord transformé par action du diazométhane pour obtenir la diazocétone N-protégée correspondante (**1**, Figure 1). Ensuite, par réarrangement de Wolff direct en présence de N,O-diméthylhydroxylamine, le N,O-diméthylhydroxamate de l'acide β-aminé N-protégé **2** est obtenu selon la méthode décrite par Limal et al. (Limal, D.; Quesnel, A.; Briand, J.P. Tet. Lett. 39, 4239-4242, 1998) (2, Figure 1). Cette étape peut être réalisée de manière plus classique en passant par l'acide β-aminé N-protégé. La réduction de cette molécule en aldéhyde est effectuée par la méthode décrite par Fehrentz et Castro (Fehrentz, J.A. & Castro, B. Synthesis 676-678, 1982). Une amination réductrice entre l'aldéhyde obtenu et une amine primaire protégée conduit à la diamine N-protégée **3**. La protection de l'amine primaire sera orthogonale à la première protection de l'acide aminé de manière à pouvoir enlever l'une des deux sélectivement. A titre d'exemple, dans le cas d'un acide aminé N-protégé par un groupe Boc, l'amine à introduire sera protégée par un groupe allyle ou benzyle. Tandis que dans le cas d'un acide aminé N-protégé par un groupe Fmoc, l'amine à introduire sera protégée par un groupe allyle. La déprotection de ce groupe permet alors d'accéder à la diamine monoprotégée **4a** ou **4b**.

A titre d'exemple, la figure 1 illustre la synthèse d'une diamine chargée de mimer la séquence dipeptidique (Ala-Val) une fois introduite dans un peptide. Cette synthèse a été réalisée selon les deux stratégies utilisées couramment en synthèse peptidique (Boc et Fmoc) pour démontrer la généralité de la voie proposée. Les rendements réactionnels sont indiqués pour chaque étape.

Le mode opératoire pour cette synthèse est décrit ci-dessous en fonction des différentes étapes :
(1) Au cours de la réaction (1), on fait réagir sur 1 équivalent d'acide aminé commercial correspondant à l'alanine N-protégée par le groupement protecteur Boc (Novabiochem, référence 04-12-0002) ou le groupement protecteur Fmoc (Novabiochem, référence 04-12-1006), 1,1 équivalents de iBuOCOCl (vendu par la société Aldrich, St Quentin Fallavier, France sous la référence 17,798-9) et 1,1 équivalents de NMM (4-méthylmorpholine, Aldrich, 40770-4), dans le THF (tétrahydrofuranne, Aldrich, 40175-7) à une concentration de 0,1 molaire en acide aminé à la température de -25°C pendant 1 heure. Le produit intermédiaire est filtré pour éliminer les sels formés.
(2) Ce produit intermédiaire réagit ensuite (étape (2)) sur du diazométhane CH₂N₂ (préparé à partir d'un précurseur Diazald vendu par la société Aldrich, référence D2,800-0 en utilisant le montage spécifique vendu par la société Aldrich sous la référence Z10,851-0) en solution dans l'éther à température ambiante pendant 2 heures. Les solvants sont évaporés à l'aide d'un évaporateur rotatif et le produit **1** est purifié par chromatographie sur silice avec un mélange acétate d'éthyle / hexane : 30/70).
(3) La réaction (3) est effectuée en mélangeant 1 équivalent du produit **1** avec 3 équivalents d'Et₃N, 0,15 équivalent de C₆H₅CO₂Ag (Aldrich, 22,727-7) dans le THF (0,1 molaire en produit **1**) puis en additionnant 1,5 équivalents de HN(OMe)Me (obtenu par neutralisation avec 2 équivalents de NEt₃ du précurseur acide vendu par Aldrich référence D16,370-8) à une température de -25°C. Le mélange réactionnel est ramené à la température ambiante pendant 2 heures. Après concentration des solvants, lavage par une solution de sulfate de potassium, séchage sur sulfate de magnésium, évaporation des solvants organiques et purification par chromatographie sur silice avec un mélange acétate d'éthyle / hexane : 50/50), le produit **2** est isolé.
(4) La réaction (4) est effectuée en faisant réagir 3 équivalents de LiAlH₄ dans le THF (Aldrich, 21776-6) à la concentration de 0,1 molaire en produit **2** à la température de -30°C pendant 1 heure. On ajoute 50 ml d'acétate d'éthyle au mélange réactionnel. L'excès d'hydrure est ensuite neutralisé par addition d'une solution aqueuse d'hydrogénosulfate de potassium, la phase organique est lavée successivement par une solution d'hydrogénocarbonate de potassium puis par une solution saturée de NaCl. La phase organique est séchée sur sulfate de magnésium, filtrée, évaporée pour conduire à l'aldéhyde correspondant.
(5) La réaction (5) est effectuée en faisant réagir 1,1 équivalents de *N*-isopropylbenzylamine (Aldrich, 13,696-4) et 1,4 équivalents de NaBH(OAc)₃ (Aldrich, 31,639-3) dans le DCE (1,2-dichloroéthane, Aldrich, 31992-9) à une concentration de 0,3 molaire en produit **2a** à température ambiante pendant 3 heures. Le mélange réactionnel est traité comme indiqué à l'étape (4) après évaporation du DCE.
(6) La réaction (6) est une hydrogénation catalytique réalisée dans le méthanol à 0,1 molaire en produit **3a** en présence de 0,1 équivalent du réactif Palladium sur lit de carbone (Aldrich, 20,569-9). Le mélange réactionnel est ensuite filtré pour éliminer le catalyseur et après évaporation du solvant, le produit **4a** est obtenu.
(7) La réaction (7) est effectuée en faisant réagir 1,1 équivalents de *N*-isopropylallylamine et 1,4 équivalents de NaBH(OAc)₃ dans le DCE à une concentration de 0,3 molaire en produit **2** à température ambiante pendant 3 heures. Le traitement effectué pour obtenir le produit **3b** ou **3c** est identique à celui de l'étape (4).
   La synthèse de la N-isopropylallylamine est la suivante.
   A une solution sous agitation d'isopropylamine (200 mmole, Aldrich 10,906-1), dans 40 ml d'eau, est additionné lentement le bromure d'allyle (100 mmole, Aldrich, A2,958-5) à température ambiante. Le mélange réactionnel est ensuite porté à reflux sur une période de 4 heures. On ajoute au mélange 10 g de soude (250 mmole) à 10°C, puis on laisse sous agitation pendant 1 heure en laissant remonter la température à 20°C. On extrait à l'éther (2 fois 30 ml) puis la phase organique est séchée sur Na₂SO₄ et le solvant est évaporé. Le résidu est distillé jusqu'au produit attendu (point d'ébullition 79°C).
(8) La réaction (8) est effectuée en faisant réagir 0,05 équivalent d'un mélange Pd(dba)₂ bis(dibenzyldèneacétone)palladium(0), vendu sous la référence 8764 par la société Lancaster, Strasbourg, France) et DPPB (1,4-bis(diphénylphosphino)butane, vendu sous la référence 8310 par la société Lancaster) dans une proportion de 1 :1 avec 2 équivalents d'acide 2-mercaptobenzoique (Aldrich, T3,320-0) dans CH₂Cl₂ à une concentration de 0,1 molaire en produit **3** à température ambiante pendant 2 heures. Après évaporation de CH₂Cl₂, le mélange réactionnel est repris dans de l'éther diéthylique, puis le composé **4b** est obtenu sous forme de chlorhydrate par précipitation en faisant buller de l'acide chlorhydrique gazeux en solution.

Les solvants sont purifiés selon les méthodes usuelles en synthèse organique (Purification of Laboratory Chemicals, 2^{nd} edition, D.D. Perrin, W.L.F. Armarego, D.R. Perrin, Pergamon Press, Oxford)

La caractérisation de ces intermédiaires par les méthodes classiques de Résonance Magnétique Nucléaire (RMN ; Bruker spectrospin, Bremen, Allemagne) et de spectrométrie de masse (MS ; MALDI TOF, Protein TOF, Bruker Spectrospin, Bremen, Allemagne) a été réalisée et les données sont en accord avec les valeurs théoriques attendues.

### Description des produits 4a et 4b :

**4a.** Solide blanc. ¹H RMN (200 MHz, CDCl₃) d(ppm) 1,21 (d, 3H, J=6,6 Hz), 1,4-1,44 (m, 6H), 1,42 (s, 9H), 1,77 (m, 1H), 2,30 (m, 1H), 2,88-3,09 (m, 2H), 3,27 (m, 1H), 3,74 (m, 1H), 4,7 (d, 1H) MALDI-TOF MS: m/z 231,2 (M+H⁺).

**4b.** Solide blanc (sel de chlorure). ¹H RMN(200 MHz, CDCl₃) d(ppm) 1,24 (d, 3H, J=6,1 Hz), 1,39-1,47 (dd, 6H, J=6,5 Hz), 1,65-2,08 (2m, 2H), 2,88-3,08 (2m, 2H), 3,26 (m, 1H), 3,71-3,92 (m, 1H), 4,20 (m, 1H), 4,39 (m, 2H), 5,23 (bb, 1H), 7,27-7,41 (m, 4H), 7,59 (d, 2H, J=6,9 Hz), 7,60 (d, 2H, J=6,8 Hz); MALDI-TOF MS: m/z 353,4 (M+H⁺).

### Exemple 2 : synthèse d'un pseudopeptide comportant le motif B carbaza selon la formule I.

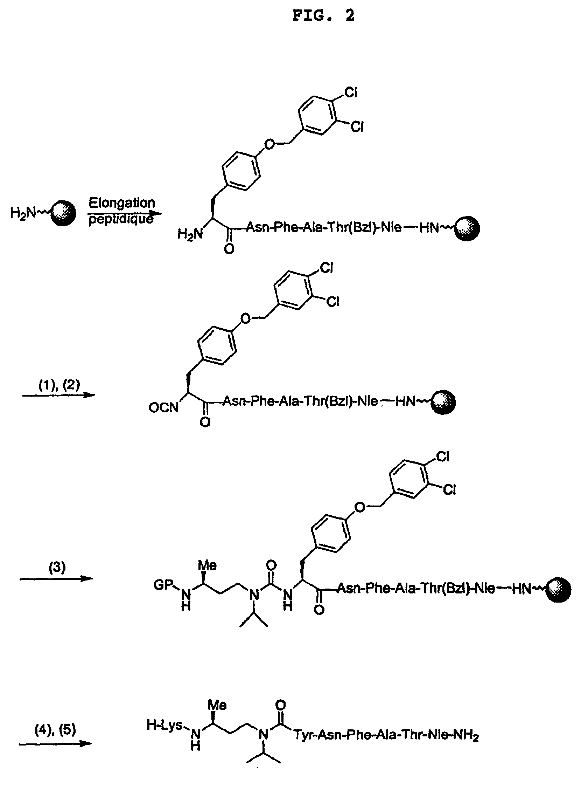

La synthèse du peptide est effectuée jusqu'au résidu tyrosine à partir d'une résine MBHA (100 micromoles de résine avec un taux de substitution de 0,63 milliéquiv. /g, référence 400373 de la société Applied Biosystems) sur un appareil Applied Biosystems (modèle 431) selon les méthodes classiques de synthèse peptidique aussi bien en utilisant la stratégie Boc ou Fmoc pour la protection des acides aminés. (voir par exemple, Synthetic peptides, a user's guide, édité par Grégory A. Grant, WH Freeman and Company, New York, 1992 ou The Practice of Peptide Synthesis, édité par M. Bodanszky et A. Bodanszky, Springer Verlag, Berlin, 1984). La molécule diaminée protégée 4a ou 4b selon les essais est ensuite couplée à une amine par l'intermédiaire d'un agent de carbonylation selon le schéma décrit dans la figure 2.

Les différentes étapes sont ci-après décrites.
1. 10 équivalents de DIEA par rapport au greffage initial de la résine (N,N-diisopropyléthylamine, Aldrich, D12,580-6) dans 2,5 ml de CH₂Cl₂ pendant 10 min à température ambiante.
2. 3,3 équivalents de triphosgène en mélange avec 10 équivalents de DIEA dans 2,5 ml de CH₂Cl₂ pendant 20 min à température ambiante. D'autres conditions en fonction de l'agent de carbonylation et du groupement protecteur GP sont données dans le tableau ci-dessous.
3. 5 équivalents du composé **4a** ou **4b** dans 2,5 ml de CH₂Cl₂ pendant 1 heure à température ambiante.
4. Déprotection de GP.
5. Elongation peptidique et coupure finale.

Tyr représente la tyrosine, Asn l'asparagine, Phe la phénylalanine, Ala l'alanine, Thr la thréonine et Nle la norleucine.

La coupure finale avec déprotection simultanée est réalisée par un mélange d'acides forts selon la procédure décrite par Fujii et al. (Fujii, N.; Otaka, A.; Ikemura, O.; Akaji, K.; Funakoshi, S.; Hayashi, Y.; Kuroda, Y.; Yajima, H. 1987 J. Chem. Soc. Chem. Commun. 274-275) ou avec de l'acide fluorhydrique dans le cas d'une synthèse en stratégie Boc. En stratégie Fmoc, la coupure est réalisée avec le réactif K (King, D.; Fileds, C.; Fileds, G. 1990 Int. J. Pept. Protein Res. 36, 255-266). La G. 1990 Int. J. Pept. Protein Res. 36, 255-266). La coupure de GP se fait selon les méthodes usuelles en synthèse peptidique.

Après purification par CLHP préparative en phase inverse, le pseudopeptide obtenu a été caractérisé par CLHP analytique et spectrométrie de masse comme décrit dans la publication Limal et al. (Limal, D.; Briand, J.P.; Dalbon, P.; Jolivet, M., 1998, J. Peptide Res. 52, 121-129).

Le tableau ci-dessous montre les différentes possibilités de synthèse ainsi que les rendements de couplage obtenus :

**Tableau**

| Stratégie de synthèse : Nature de GP | Réactif de carbonylation Etape (2) | Temps de réaction pour la diamine (heure) | Rendement total de synthèse du pseudopeptide après purification CLHP (%) |
|---|---|---|---|
| Boc | Carbo diimidazole | 1 | 6 |
| Boc | Carbo diimidazole | 12 | 30 |
| Boc | Carbo diimidazole | 72 | 35 |
| Boc | Triphosgène | 1 | 35 |
| Boc | Triphosgène | 12 | 32 |
| Boc | Triphosgène | 1 | 40 |
| Fmoc | Triphosgène | 12 | 25 |

Temps de rétention en CLHP du pseusopeptide : 11min 88. MALDI-TOF MS: m/z 1012,05 (M+H⁺) en accord avec le poids théorique.

### Exemple 3 : synthèse d'un pseudopeptide comportant le motif B carbaza selon la formule II.

La molécule-clé pour la synthèse du pseudopeptide est une diamine monoprotégée et il est donc naturel de pouvoir l'introduire dans la synthèse par l'une ou l'autre de ses fonctions amines. Pour cela, on a protégé à nouveau l'extrémité comportant l'amine secondaire du composé 4a à l'aide d'un groupement Fmoc et déprotégé l'extrémité protégé par le groupe Boc. La molécule obtenue a été introduite sur support solide de la même manière que précédemment. On obtient ainsi la molécule représentée ci-dessous avec une masse identique au composé pseudopeptidique précédent mais avec un temps de rétention différent. 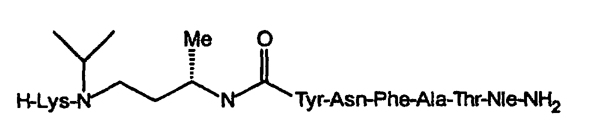 Temps de rétention en CLHP : 11min 55. (conditions CLHP décrites dans l'exemple 2)
MALDI-TOF MS: m/z 1012,05 (M+H⁺)

## Revendications

1. Pseudopeptide d'au moins 6 acides aminés comprenant au moins un motif choisi parmi les motifs de formules générales (I) et/ou (II) : 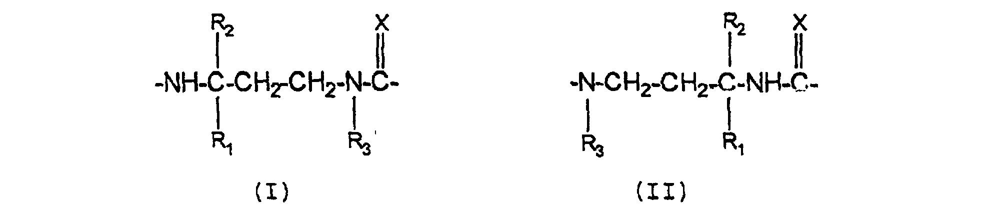 dans lesquelles :
R₁, R₂ et R₃ représentent chacun indépendamment l'un de l'autre une chaîne latérale d'acides aminés et peuvent être identiques ou différents,
X représente un atome d'oxygène ou de soufre.

2. Pseudopeptide selon la revendication 1 d'une taille d'au moins 9 acides aminés.

3. Pseudopeptide selon la revendication 1 ou 2, **caractérisé en ce que** X représente un atome d'oxygène.

4. Pseudopeptide selon l'une quelconque des revendications 1 à 3, **caractérisé en ce que** R₂ représente un atome d'hydrogène.

5. Procédé de synthèse d'un pseudopeptide selon l'une quelconque des revendications 1 à 4, **caractérisé en ce que** l'on effectue un couplage d'une diamine monoprotégée de formule générale IIIa ou IIIb 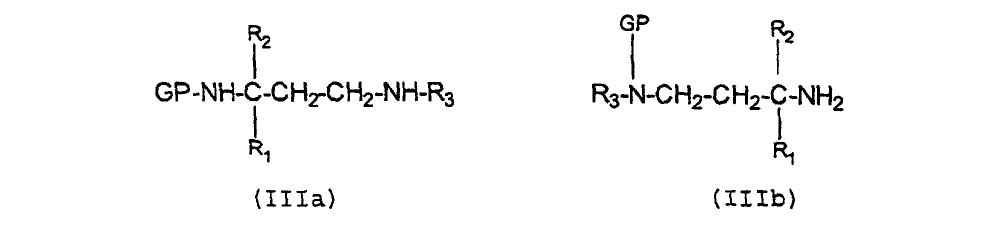 dans lesquelles, R₁, R₂ et R₃ représentent chacun indépendamment l'un de l'autre une chaîne latérale d'acides aminés et peuvent être identiques ou différents, GP représente un groupement protecteur de fonction amine
avec une amine en présence d'un agent de carbonylation.

6. Procédé selon la revendication 5, **caractérisé en ce que** GP est un groupement Boc, Fmoc, Cbz ou Alloc.

7. Procédé selon la revendication 5 ou 6, **caractérisé en ce que** l'agent de carbonylation est choisi parmi le N,N'-carbonyldiimidazole et le triphosgène.

8. Réactif pour la détection d'une pathologie associée à la présence de protéines endogènes ou exogènes, **caractérisé en ce qu'**il comprend à titre de substance réactive au moins un pseudopeptide selon l'une quelconque des revendications 1 à 4.

9. Réactif selon la revendication 8, **caractérisé en ce que** le pseudopeptide est marqué par un traceur ou la biotine.

10. Réactif selon les revendications 8 et 9, **caractérisé en ce que** la taille du pseudopeptide est au moins de 12 acides aminés.

11. Kit de détection d'une pathologie associée à la présence de protéines endogènes ou exogènes **caractérisé en ce qu'**un réactif selon l'une quelconque des revendications 8 à 10, est fixé sur un support solide immunologiquement compatible avec ledit réactif.

12. Procédé de détection et/ou de dosage de molécules biologiques présentes dans un échantillon dans lequel on utilise le réactif selon l'une quelconque des revendications 8 à 10, pour former un complexe immun avec lesdites molécules biologiques si elles sont présentes dans l'échantillon.

13. Procédé de détection selon la revendication 12 **caractérisé en ce que** les molécules biologiques sont des anticorps.

14. Procédé pour la détection et/ou le dosage d'un antigène présent dans un échantillon par une technique de compétition dans lequel on met en contact simultanément ou en deux étapes ledit échantillon avec une quantité prédéterminée d'anticorps dirigé contre un partie de l'antigène et une quantité prédéterminée d'un réactif selon l'une quelconque des revendications 8 à 10, et on détermine la présence et/ou la quantité d'antigène présent dans ledit échantillon.

15. Procédé pour la détection et/ou le dosage d'un anticorps présent dans un échantillon par une technique de compétition dans lequel on met en contact simultanément ledit échantillon avec une quantité prédéterminée d'antigène dont au moins une partie est reconnue par ledit anticorps et une quantité prédéterminée d'un réactif selon l'une quelconque des revendications 8 à 10, et on détermine la présence et/ou la quantité d'anticorps présent dans ledit échantillon.

16. Anticorps monoclonal ou polyclonal susceptible d'être qu'obtenu par immunisation d'un animal avec au moins un pseudopeptide selon l'une quelconque des revendications 1 à 4.

17. Anti-idiotype susceptible d'être obtenu par immunisation d'un animal avec au moins un anticorps selon la revendication 16.

18. Composition thérapeutique active, notamment composition immunothérapeutique active, **caractérisée en ce qu'**elle comprend à titre de principe actif au moins un pseudopeptide selon l'une quelconque des revendications 1 à 4, un anticorps selon la revendication 16, ou un anti-idiotype selon la revendication 17, ledit principe actif étant éventuellement sous la forme d'un conjugué ou d'un excipient pharmaceutiquement acceptable.

## Patentansprüche

1. Pseudopeptid mit zumindest 6 Aminosäuren, welches zumindest ein Motiv aufweist, welches ausgewählt ist aus den Motiven der allgemeinen Formeln (I) und (II) 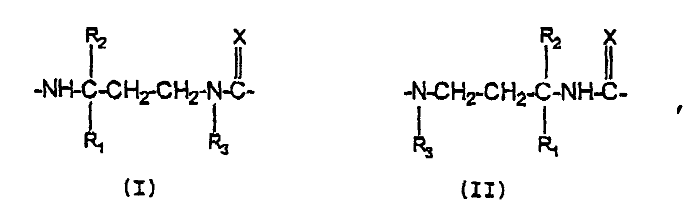 in welchen:
R₁, R₂ und R₃ jeweils unabhängig voneinander eine Seitenkette der Aminosäuren darstellen und entweder gleich oder unterschiedlich sein können, und
X ein Sauerstoff- oder Schwefelatom ist.

2. Pseudopeptid nach Anspruch 1, das eine Länge von zumindest 9 Aminosäuren aufweist.

3. Pseudopeptid nach Anspruch 1 oder 2, **dadurch gekennzeichnet, daß** x ein Sauerstoffatom darstellt.

4. Pseudopeptid nach einem der Ansprüche 1 bis 3, **dadurch gekennzeichnet, daß** R₂ ein Wasserstoffatom darstellt.

5. Verfahren zur Synthese eines Pseudopeptids nach einem der Ansprüche 1 bis 4, **dadurch gekennzeichnet, daß** ein einfach-geschütztes Diamin der allgemeinen Formel IIIa oder IIIb 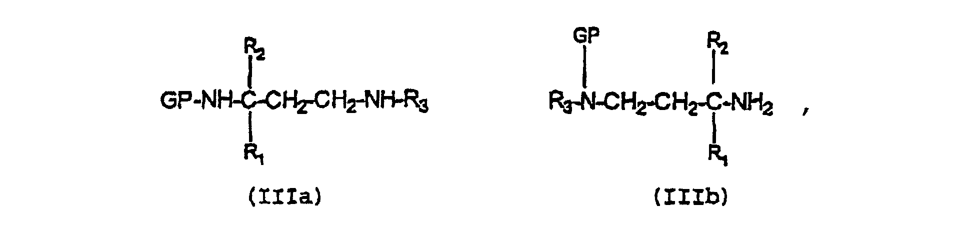 in welchen R₁, R₂ und R₃ jeweils unabhängig voneinander eine Seitenkette der Aminosäuren darstellen und entweder gleich oder unterschiedlich sein können, und GP eine Gruppe mit Funktion einer Amin-Schutzgruppe darstellt,
unter Anwesenheit eines Carbonylierungs-Mittels mit einem Amin verknüpft wird.

6. Verfahren nach Anspruch 5, **dadurch gekennzeichnet, daß** GP eine Boc-, Fmoc-, Cbz- oder Alloc-Gruppe ist.

7. Verfahren nach Anspruch 5 oder 6, **dadurch gekennzeichnet, daß** das Carbonylierungsmittel aus N,N'-Carbonyldiimidazol und Triphosgen ausgewählt ist.

8. Reagens für die Detektion eines Krankheitsbildes, welches mit dem Vorkommen von endogenen oder exogenen Proteinen verbunden ist, **dadurch gekennzeichnet, daß** dieses als reaktive Substanz zumindest ein Pseudopeptid nach einem der Ansprüche 1 bis 4 aufweist.

9. Reagens nach Anspruch 8, **dadurch gekennzeichnet, daß** das Pseudopeptid durch einen Indikator oder Biotin markiert ist.

10. Reagens nach Anspruch 8 oder 9, **dadurch gekennzeichnet, daß** das Pseudopeptid eine Länge von zumindest 12 Aminosäuren aufweist.

11. Kit zur Detektion eines Krankheitsbildes, welches mit dem vorkommen von endogenen oder exogenen Proteinen verbunden ist, **dadurch gekennzeichnet, daß** ein Reagens nach einem der Ansprüche 8 bis 10 auf einem festen Träger fixiert ist, welcher mit dem Reagens immunologisch kompatibel ist.

12. Verfahren zur Detektion und/oder Dosierung von biologischen Molekülen, welche in einer Probe vorliegen, nach welchem das Reagens nach einem der Ansprüche 8 bis 10 zur Bildung eines immunologischen Komplexes mit den biologischen Molekülen verwendet wird, wenn diese in der Probe vorliegen.

13. Verfahren zur Detektion nach Anspruch 12, **dadurch gekennzeichnet, daß** die biologischen Moleküle Antikörper sind.

14. Verfahren zur Detektion und/oder Dosierung eines Antigens, welches in einer Probe vorliegt, mittels einer Kompetitionstechnik, nach welchem die Probe entweder gleichzeitig oder in zwei Schritten mit einer bestimmten Menge an einem Antikörper, welcher gegen einen Teil des Antigens gerichtet ist, und mit einer bestimmten Menge eines Reagens nach einem der Ansprüche 8 bis 10 in Kontakt gebracht wird, und das Vorliegen und/oder die Menge eines Antigens bestimmt wird, welches in der Probe vorliegt.

15. Verfahren zur Detektion und/oder Dosierung eines Antikörpers, welcher in einer Probe vorliegt, mittels einer Kompetitionstechnik, nach welchem die Probe gleichzeitig mit einer bestimmten Menge an Antigen, von welchem zumindest ein Teil durch den Antikörper erkannt wird, und mit einer bestimmten Menge eines Reagens nach einem der Ansprüche 8 bis 10 in Kontakt gebracht wird, und das Vorliegen und/oder die Menge eines Antikörpers bestimmt wird, welcher in der Probe vorliegt.

16. Monoklonaler oder polyklonaler Antikörper, welcher durch Immunisierung eines Tieres mit zumindest einem Pseudopeptid nach einem der Ansprüche 1 bis 4 erhältlich ist.

17. Anti-Idiotyp, welcher durch Immunisierung eines Tieres mit zumindest einem Pseudopeptid nach einem der Ansprüche 1 bis 4 erhältlich ist.

18. Therapeutisch aktive Zusammensetzung, insbesondere immuntherapeutisch aktive Zusammensetzung, **dadurch gekennzeichnet, daß** diese als Wirkstoff zumindest ein Pseudopeptid nach einem der Ansprüche 1 bis 4 aufweist, ferner einen Antikörper nach Anspruch 16 oder einen Anti-Idiotyp nach Anspruch 17, wobei der Wirkstoff gegebenenfalls in Form eines Konjugats oder eines pharmazeutisch akzeptablen Exzipienten vorliegen kann.

## Claims

1. Pseudopeptide of at least 6 amino acids comprising at least one motif chosen from the motifs of general formulae (I) and/or (II): 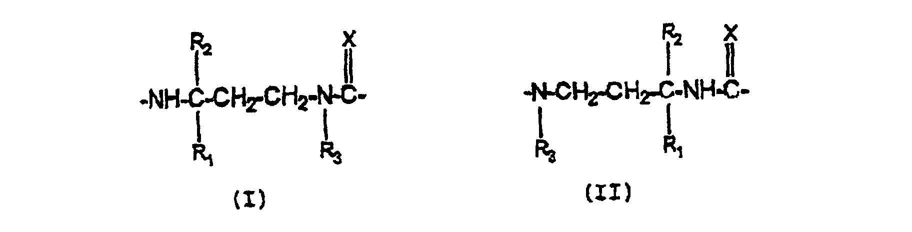 in which:
R₁, R₂ and R₃ each represent, independently of one another, an amino acid side chain and may be identical or different,
X represents an oxygen or sulphur atom.

2. Pseudopeptide according to Claim 1, which is at least 9 amino acids in size.

3. Pseudopeptide according to Claim 1 or 2, **characterized in that** X represents an oxygen atom.

4. Pseudopeptide according to any one of Claims 1 to 3, **characterized in that** R₂ represents a hydrogen atom.

5. Method for synthesizing a pseudopeptide according to any one of Claims 1 to 4, **characterized in that** a monoprotected diamine of general formula IIIa or IIIb 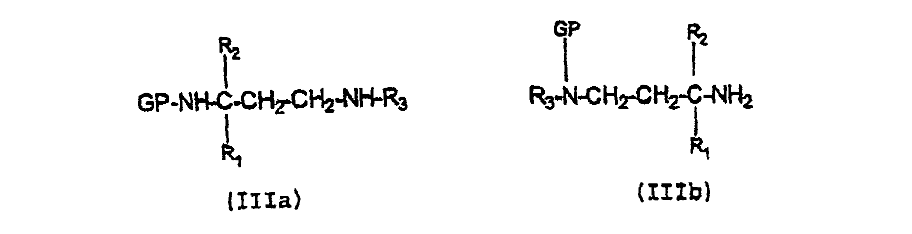 in which R₁, R₂ and R₃ each represent, independently of one another, an amino acid side chain and may be identical or different, and GP represents a protective group for an amine function,
is coupled with an amine in the presence of a carbonylating agent.

6. Method according to Claim 5, **characterized in that** GP is a Boc, Fmoc, Cbz or Alloc group.

7. Method according to Claim 5 or 6, **characterized in that** the carbonylating agent is chosen from N,N'-carbonyldiimidazole and triphosgene.

8. Reagent for detecting a pathology associated with the presence of endogenous or exogenous proteins, **characterized in that** it comprises, as. reactive substance, at least one pseudopeptide according to any one of Claims 1 to 4.

9. Reagent according to Claim 8, **characterized in that** the pseudopeptide is labelled with a tracer or biotin.

10. Reagent according to Claims 8 and 9, **characterized in that** the pseudopeptide is at least 12 amino acids in size.

11. Kit for detecting a pathology associated with the presence of endogenous or exogenous proteins, **characterized in that** a reagent according to any one of Claims 8 to 10 is attached to a solid support which is immunologically compatible with said reagent.

12. Method for detecting and/or assaying biological molecules present in a sample, in which the reagent according to any one of Claims 8 to 10 is used to form an immunocomplex with said biological molecules if they are present in the sample.

13. Detection method according to Claim 12, **characterized in that** the biological molecules are antibodies.

14. Method for detecting and/or assaying an antigen present in a sample using a competition technique in which said sample is brought into contact, simultaneously or in two steps, with a predetermined amount of antibody directed against part of the antigen and a predetermined amount of a reagent according to any one of Claims 8 to 10, and the presence and/or amount of antigen present in said sample is determined.

15. Method for detecting and/or assaying an antibody present in a sample using a competition technique in which said sample is brought into contact simultaneously with a predetermined amount of antigen, at least part of which is recognized by said antibody, and a predetermined amount of a reagent according to any one of Claims 8 to 10, and the presence and/or amount of antibody present in said sample is determined.

16. Monoclonal or polyclonal antibody which can be obtained by immunizing an animal with at least one pseudopeptide according to any one of Claims 1 to 4.

17. Anti-idiotype which can be obtained by immunizing an animal with at least one antibody according to Claim 16.

18. Active therapeutic composition, in particular active immunotherapeutic composition, **characterized in that** it comprises, as active principle, at least one pseudopeptide according to any one of Claims 1 to 4, an antibody according to Claim 16 or an anti-idiotype according to Claim 17, said active principle optionally being in the form of a conjugate or of a pharmaceutically acceptable excipient.
